Europäisches Patentamt

European Patent Office

Office européen des brevets

(11) Publication number: **0 144 057 B1**

# EUROPEAN PATENT SPECIFICATION

(45) Date of publication of patent specification: **03.04.91**   (51) Int. Cl.⁵: **G01N 27/417**

(21) Application number: **84114331.6**

(22) Date of filing: **27.11.84**

(54) Apparatus for measuring oxygen concentration and method of producing the appartus.

(30) Priority: **28.11.83 JP 223941/83**

(43) Date of publication of application:
**12.06.85 Bulletin 85/24**

(45) Publication of the grant of the patent:
**03.04.91 Bulletin 91/14**

(84) Designated Contracting States:
**DE FR GB**

(56) References cited:
**EP-A- 0 011 530      EP-A- 0 020 938
EP-A- 0 035 400      EP-A- 0 104 636
EP-A- 0 140 295      FR-A- 2 334 101
US-A- 4 381 224**

(73) Proprietor: **HITACHI, LTD.**
**6, Kanda Surugadai 4-chome
Chiyoda-ku, Tokyo 100(JP)**

(72) Inventor: **Suzuki, Seikoh**
**3705 Kanaicho
Hitachiota-shi(JP)**
Inventor: **Miki, Masayuki**
**38-480 Yakushidai-Apartment House
Kosunacho Katsuta-shi(JP)**
Inventor: **Sasayama, Takao**
**7-14-11 Kanesawacho
Hitachi-shi(JP)**
Inventor: **Suzuki, Toshitaka**
**28-32 Ishinazakacho-1-chome
Hitachi-shi(JP)**
Inventor: **Ueno, Sadayasu**
**847-33 Ichige
Katsuta-shi(JP)**

(74) Representative: **Patentanwälte Beetz sen. -
Beetz jun. Timpe - Siegfried - Schmitt-
Fumian
Steinsdorfstrasse 10
W-8000 München 22(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to an apparatus for measuring oxygen concentration and, more particularly, to an oxygen concentration measuring apparatus for measuring the oxygen concentration in exhaust gas emitted from an internal combustion engine. The invention relates also to a method of producing the oxygen concentration measuring apparatus.

Modern internal combustion engines having an air-fuel ratio controller employ an air-fuel ratio sensor adapted to measure the oxygen concentration in the exhaust gas.

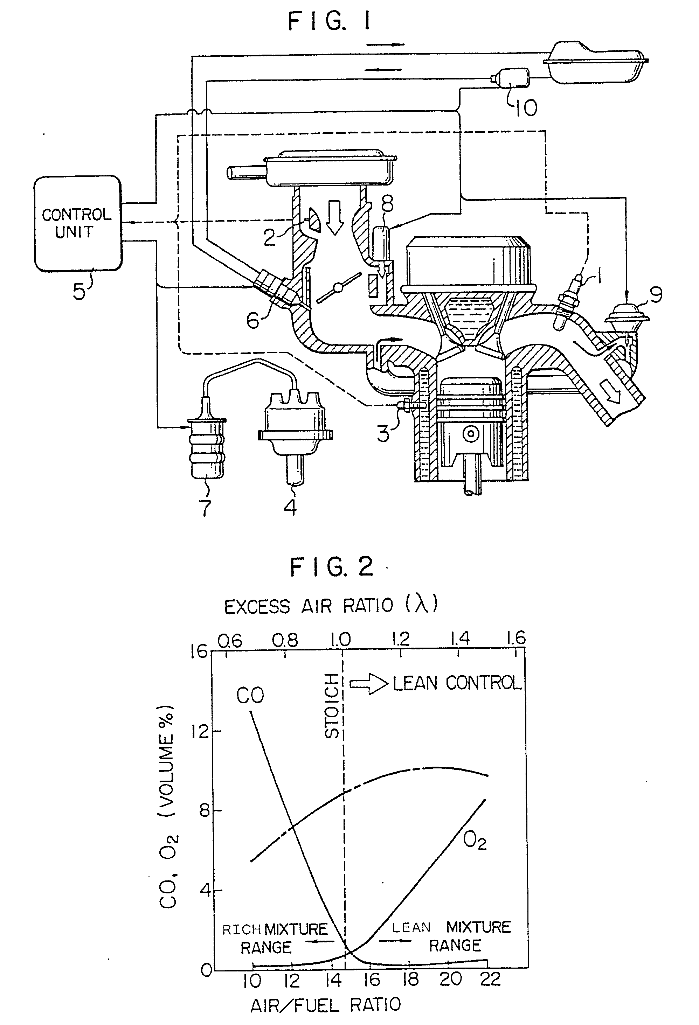

Referring to Fig. 1, a typical air-fuel ratio controller has a control unit 5 adapted to receive various information concerning the engine operation delivered by sensors such as an air-fuel ratio sensor 1, air-flow sensor 2, water-temperature sensor 3, and crank-position sensor 4. In response to these information, the control unit 5 controls the operation of various devices such as a fuel injector 6, ignition coil 7, idle speed control valve 8, exhaust gas recirculation control valve 9 and a fuel pump 10.

The air-fuel ratio sensor incorporated in this air-fuel ratio controller could measure only the stoichiometric air-fuel ratio, and the control of the air-fuel ratio is made to maintain the air-fuel ratio at the stoichiometric level, in order to reduce the noxious exhaust emissions. Fig. 2 shows how the concentrations of oxygen and carbon monoxide, as well as the combustion efficiency, are changed in relation to the air-fuel ratio. In the conventional air-fuel ratio controller, the control is made such as to maintain the stoichiometric air-fuel ratio (A/F = 14.7 excess-air ratio $\lambda$ = 1), except the case where a specifically large power is required as in the case of acceleration in which a mixture richer than the stoichiometric one is set.

On the other hand, it is well known that the maximum combustion efficiency is obtained by the leaner side of the stoichiometric level as will be seen from Fig. 2. From this point of view, it is desirable to control the air-fuel ratio such that the internal combustion engine operates on the leaner side of the stoichiometric air-fuel ratio. Such a control requires an air-fuel ratio sensor, i.e., an oxygen concentration measuring apparatus, capable of accurately detecting the air-fuel ratio.

Oxygen concentration measuring apparatus have been known in which the oxygen concentration in exhaust emission is measured by making use of an oxygen pumping phenomenon exibited by an oxygen ion-conductive solid electrolyte. For instance, Japanese Patent Laid-Open Nos. 69690/1977 and 72286/1977, and US-A- 4, 381, 224 disclose oxygen concentration measuring apparatus as generally shown in Fig. 3.

More specifically, this oxygen concentration measuring apparatus is composed of an oxygen ion-conductive solid electrolyte 11 of zirconia, first and second electrodes 12 and 13 formed on both sides of the solid electrolyte 11, a cover member 14 surrounding the first electrode 12, a gas diffusing aperture 15 and a diffusion chamber 16. In operation, a voltage E is applied between two electrodes such that the first electrode 12 serve as a cathode, and the quantity of flow of oxygen ions in the solid electrolyte is measured by an ampere meter 18.

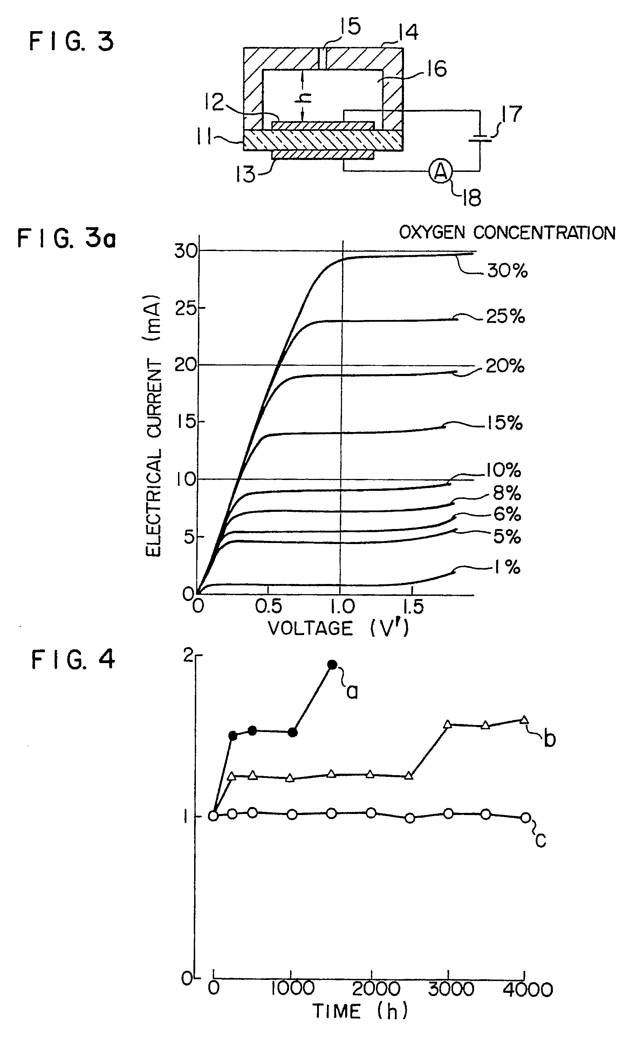

In the actual use of the oxygen concentration measuring apparatus of the type mentioned above, the sensor head composed of the electrolyte 11, electrodes 12, 13, cover member 14 and the gas diffusion aperture 15 is exposed to the hot gas to be examined (temperature 600 to 1000°C) and the voltage applied between both electrodes 12 and 13, is gradually increased. The increase of the voltage causes a corresponding increase in the electric current flowing between the electrodes 12 and 13 but this increase in the electric current is saturated and maintained constant when the voltage has been increased to a certain level. The relationship between the electric current and the voltage is shown in Fig. 3a.

This can be attributable to a phenomenon called "oxygen pumping phenomenon" in which the oxygen introduced into the diffusion chamber 16 through the diffusion aperture 15 is reduced into oxygen ions by the first electrode 12, and the ions after being transmitted through the solid electrolyte 11 is oxidized again into oxygen upon reaching the second electrode. The saturation of the electric current is attributable to a restriction imposed by the diffusion aperture 15. Namely, the supply of the oxygen to be reduced into oxygen ions by the first electrode 12 is limited by the diffusion aperture 15.

According to this arrangement, when the voltage E is approximately 1 V, the electric current Ip changes substantially in proportion to the concentration of oxygen in the gas to be examined, as will be understood from the following formula (1).

$$I_F = \frac{4FDS}{RT\ell} PO_2 \quad \cdots \cdots \cdots \cdots (1)$$

where F represents the Faraday constant, D represents the diffusion constant of oxygen, R represents

EP 0 144 057 B1

the gas constant, T represents absolute temperature, S represents the cross-sectional area of the gas diffusion aperture, $\ell$ represents the length of the gas diffusion aperture and $PO_2$ represents the partial pressure of oxygen.

Representing the internal volume of the diffusion chamber by $V_r$, the response characteristic of this sensor is approximately given by the following formula (2).

$$\tau = \frac{V_r \ell}{DS} \quad \ldots\ldots\ldots\ldots\ldots\ldots \quad (2)$$

The conventional oxygen concentration measuring apparatus shown in Fig. 3, however, suffers from the following disadvantages.

The cover member 14 is fixed to the zirconia solid electrolyte by bonding through a bond such as a glass frit, ceramics, glue or the like, so that the cover member 14 is liable to come off from the solid electrolyte 11 by a thermal impact, resulting in a secular change in the sensing performance.

Fig. 4 shows the result of an experiment conducted to examine how the sensing characteristic is changed in relation to time. More specifically, this Figure shows how the output current Ip is changed in relation to time from a base level Ip = 1 obtained in the initial stage at temperature of 800°C when the oxygen concentration is 10%, for each of the samples a , b and c . The sample a in which a ceramics bond is used showed a drastic increase of the output current Ip in quite a short time of use due to the separation of the cover member from the solid electrolyte which caused an increase in the gas permeation cross-sectional area equivalent to an increase in the cross-sectional area of the gas diffusion aperture 15. The sample a , therefore, is not usable practically.

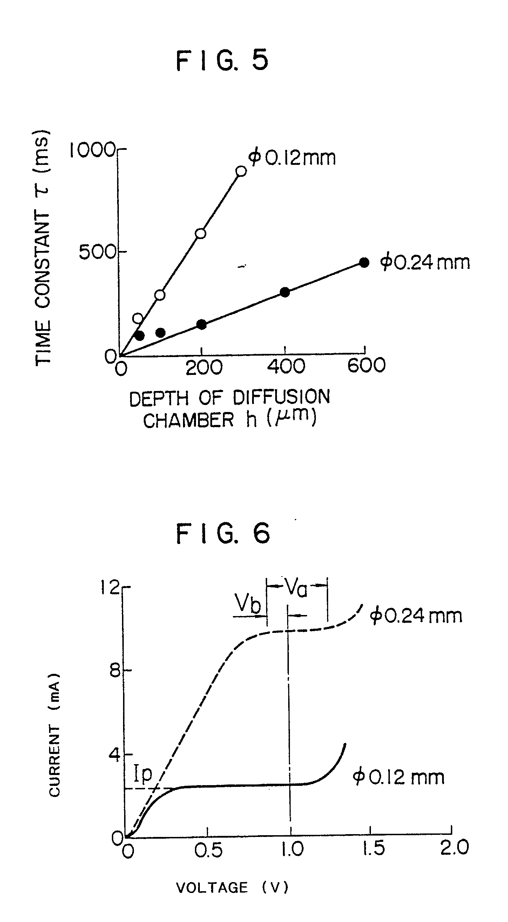

The use of bond imposes another problem concerning the response characteristic as will be explained hereinunder with reference to Fig. 5.

Fig. 5 shows the time constant $\tau$, as measured with samples having gas-diffusion aperture diameters of 0.12 mm and 0.14 mm, while varying the depth h of the diffusion chamber. In both cases, the diffusion chamber had equal cross-sectional area of 16 mm² (4 mm high and 4 mm wide) and an equal diffusion aperture length of 0.4 mm. For attaining a high response characteristic of the control, it is desirable that the time constant is on the order of several tens of microseconds (ms) and should not exceed 100 ms. For obtaining such a small time constant, it is necessary that the depth h of the diffusion chamber 16 is not greater than 50 μm which, however, is extremely difficult to attain because the thickness of the bond is not negligible. Consequently, the practically obtainable time constant is, at the smallest, much greater than the desired value. From formula (2) mentioned before, it is readily understood that the time constant can be decreased as the diameter of the gas diffusion aperture 15 is increased. An experiment made by the inventors, however, proved a fact that the effect of improvement in the response characteristic by the increase of the diameter of the diffusion aperture 15 is gradually decreased when the depth h of the diffusion chamber 16 approaches the diameter of the gas diffusion aperture 15. This may be attributed to a fact that the diffusion aperture 15 becomes close to the first electrode 12 as the depth h of the diffusion chamber 16 is reduced so that the diffusion along the surface of the solid electrolyte is limited to an insufficient level.

Under these circumstances, the inventors attempted to improve the response characteristics by further increasing the diameter of the gas diffusion aperture 15. However, as will be seen from Fig. 6 showing an E-I characteristic of the test sensor, it proved that there is a practical limit also in this method. More specifically, Fig. 6 shows the oxygen pumping current I as measured by the ampere meter 18 in relation to the voltage E applied between the first and the second electrodes 12 and 13, in the case of that the temperature of the gas to be examined is 800°C and the oxygen concentration is 10%, for each of test sensors having diffusion aperture diameters of 0.12 mm and 0.24 mm. As will be understood from this Figure, the pump current I is gradually increased as the voltage E applied between both electrodes becomes higher, and is saturated at a constant critical current Ip. The operation of this sensor relies upon the detection of this critical current Ip. A further increase in the voltage E applied causes a change from the ion-conductive state to an electron-conductive state of the zirconia solid electrolyte 11, thus allowing a drastic increase of the electric current. Needless to say, high precision of oxygen concentration measurement cannot be expected in this region where the electric current is creased drastically. On the other hand, the width Va of the region where the oxygen pumping current I is saturated and mantained substantially constant is increased as the diameter of the gas-diffusion aperture is increased, i.e., as the critical current Ip is increased. This means that, when the voltage applied between two electrodes is maintained at a constant level of 1V, the margin voltage width Vb for the detection of the saturated electric current Ip becomes

3

smaller as the diameter of the gas diffusion aperture 15 is increased. Since this margin Vb tends to become smaller due to a secular change, it is not allowed to make the margin Vb unlimitedly narrow from the view point of reliability. The improvement in the response characteristic by the increase of the diameter of the gas diffusion aperture 15 is limited also by this fact. As shown in Fig. 6, the sensor is designed to exhibit a rather small critical current Ip. This is because the catalytic activities in the first and second electrodes 12 and 13 are practically limited. For information the data as shown in Fig. 6 has been obtained when the area of the electrode is 10 mm². The value of the oxygen pumping current which can be converted per unit area was about 1 mA/mm².

Thus, the conventional oxygen concentration measuring apparatus encountered various problems concerning the durability and response characteristics.

EP-A-0 011 530 discloses an oxygen concentration measuring apparatus comprising a diffusion chamber formed between a zirconia solid electrolyte having a pair of electrodes formed on each side thereof and a cover member encasing one of said electrodes, which diffusion chamber is provided with a gas diffusion groove located laterally of the encased electrode between the solid electrolyte and the cover member.

EP-A-0 140 295 of earlier priority date discloses a similar oxygen concentration measuring apparatus as EP-A-0 011 530 and describes, in addition, the feature that the solid electrolyte and the cover member are directly bonded and integrated with each other, i.e. by an organic binder burning method.

An object of the invention is to provide an oxygen concentration measuring apparatus which does not suffer from small secular change in the output characteristics while ensuring a good response characteristics, and a method of producing said apparatus.

To this end, according to the invention, the undesirable secular change in the output characteristics is avoided by a strong fixing of the cover member to the zirconia solid electrolyte attained by a process by laminating a zirconia solid electrolyte in the form of a green sheet and a cover member while they are in the unfired state, heating and press-bonding the zirconia solid electrolyte and the cover member at a temperature of about 200° C, and effecting a firing to integrate the cover member to the zirconia solid electrolyte without using any adhesive or bond.

The oxygen concentration measuring apparatus according to the present invention comprises: a zirconia solid electrolyte, a pair of electrodes formed on each side of said zirconia solid electrolyte and connected to a supply of electric current, a cover member provided on said solid electrolyte and encasing one of said electrodes, and a diffusion chamber formed between said solid electrolyte and said cover member and provided with at least one gas diffusion groove, characterized in that the solid electrolyte and the cover member are directly bonded and integrated with each other, and that two gas diffusion grooves are provided at both lateral sides of the encased electrode between the solid electrolyte and the cover member.

The method of producing an oxygen concentration measuring apparatus according to the present invention comprises the steps of: forming a first electrode and a second electrode on each side of a plate of a solid electrolyte, and attaching a cover member encasing said first electrode and forming a diffusion chamber between said solid electrolyte and said cover member, which diffusion chamber is provided with a gas diffusion orifice, characterized in that after forming said first and second electrodes by printing, a layer of an organic binder is formed on one side of said solid electrolyte so as to cover said first electrode; a sheet-like cover member is laminated on the same side of said solid electrolyte as said first electrode thus forming a laminated structure; said laminated structure is subjected to a heat- and press-bonding at a temperature of about 200° C; the temperature of said laminated structure is raised at a controlled rate to gradually burn said binder so as to remove said binder thus forming a diffusion chamber on said first electrode between said cover member and said solid electrolyte, said diffusion chamber being provided with two gas diffusion grooves located at both lateral sides of said first electrode; and said laminated structure is fired at a temperature of about 1500° C so as to sinter said solid electrolyte and said cover member uniformly into one body.

The invention will be more fully described hereinunder with reference to the accompanying drawings.

Fig. 1 is a schematic illustration of an air-fuel ratio controller of an internal combustion engine;

Fig. 2 is an illustration showing how the contents of the exhaust gas and the combustion efficiency are changed in relation to the air-fuel ratio;

Fig. 3 is an illustration of a conventional oxygen concentration measuring apparatus;

Fig. 3a is a chart showing the current-voltage characteristics of the oxygen concentration measuring apparatus shown in Fig. 3;

Fig. 4 is a chart showing the secular change of the operation characteristics;

Fig. 5 is a chart showing the response characteristics of the conventional oxygen concentration measuring apparatus;

Fig. 6 is a chart showing the E-I characteristic of a test sensor;

Figs. 7 to 9 are illustrations of an organic binder burning process;

Figs. 10 and 11 show the construction of an embodiment of the oxygen concentration measuring apparatus in accordance with the invention; and

Figs. 12 and 13 are illustrations of another embodiment of the invention.

A description will be made first as to the organic binder burning process which is used in the formation of the diffusion chamber and diffusion groove in the oxygen concentration measuring apparatus of the invention.

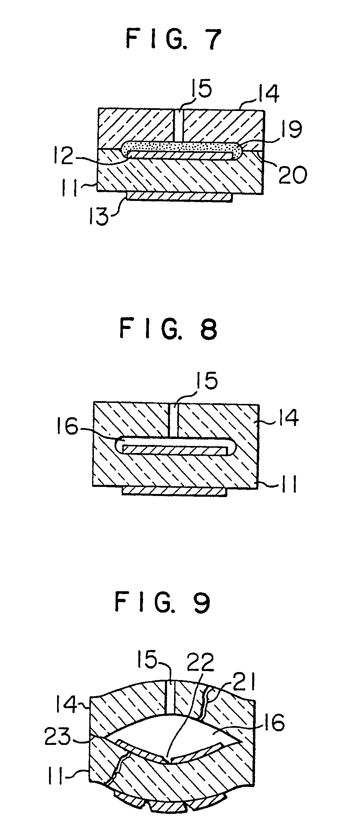

Fig. 7 shows how the organic binder burning process is applied to the production of an oxygen concentration measuring apparatus of the conventional construction. A first electrode 12 and a second electrode 13 in the form of thick films are formed by printing on both sides of a zirconia solid electrolyte 11 in the form of a green sheet. Then, an organic binder 19 containing carbon and other components (thickness less than several tens of $\mu$m) is formed by printing on the electrode 12. A cover member 14 in the form of a green sheet having a gas diffusion aperture 15 formed therein by drilling is laminated to the electrolyte 11 through the intermediary of the organic binder 19. The laminated structure thus obtained is then subjected to a heat- and press-bonding conducted by means of a press at a temperature of about 200°C. Then, the temperature is raised at a rate not greater than several degrees per minute so that the organic binder is gradually burnt and removed to leave a room corresponding to the diffusion chamber between two layers. The gases produced as a result of the burning of the organic binder is discharged to the outside through the gas diffusion aperture 15. The laminated structure is then subjected to a firing conducted at a temperature of about 1500°C, so that the solid electrolyte and the cover member 14 are uniformly sintered into one body. Namely, the boundary 20 between the zirconia solid electrolyte and the cover member is extinguished to allow a direct bonding of these two layers, while leaving a diffusion chamber 16 of a configuration substantially conforming with the form of the organic binder immediately after the heat- and press-bonding. Taking into the thermal stress into consideration, it is necessary that the cover member 14 is made of a material having a thermal expansion coefficient substantially same as that of the zirconia solid electrolyte 11. Preferably, the cover member 14 itself is made of the zirconia solid electrolyte. Fig. 8 schematically shows an example in which the cover member 14 is made of the zirconia solid electrolyte.

The cross-sectional area of the gas diffusion aperture 15 is as small as 1/1000 or less of the cross-sectional area of the diffusion chamber 16, so that the rate of temperature rise during the burning of the organic binder has to be very small because otherwise the laminated structure is inflated due to a rise in the internal gas pressure in the portion corresponding to the diffusion chamber 16 during burning, resulting in the formation of pin-holes 21 and cracking 22 in the electrodes, while reducing the area of direct bond between the solid electrolyte 11 and the cover member 14 to allow an easy cracking by thermal impact, as shown in an exaggerated manner in Fig. 9.

It would be possible to suppress any exessive rise of the internal gas pressure by increasing the number of the gas diffusion apertures 15 and the cross-sectional area thereof. In this case, however, it is not allowed to increase the number of the apertures and cross-sectional area unlimitedly because the capacity of the oxygen pumping function of the electrodes is limited.

Thus, when the organic binder burning method is applied to the conventional oxygen concentration measuring apparatus, it is quite important to stabilize the conditions or factors such as the rate of temperature rise during the burning, which is generally difficult to attain.

The sample b mentioned before in connection with Fig. 4 is an oxygen concentration sensor preapred by the methods explained above. Although this sample b exhibits a secular-change characteristics considerably improved as compared with the sample a , this sample b is still unsatisfactory from a view point of practicability. The secular change of this sample b is attributable to generation of micro-cracks in the bonding area and other portions due to a thermal impack as a result of an excesive rise of the internal gas pressure during the burning of the organic binder 19.

These problems, however, are overcome by the present invention as will be understood from the following description.

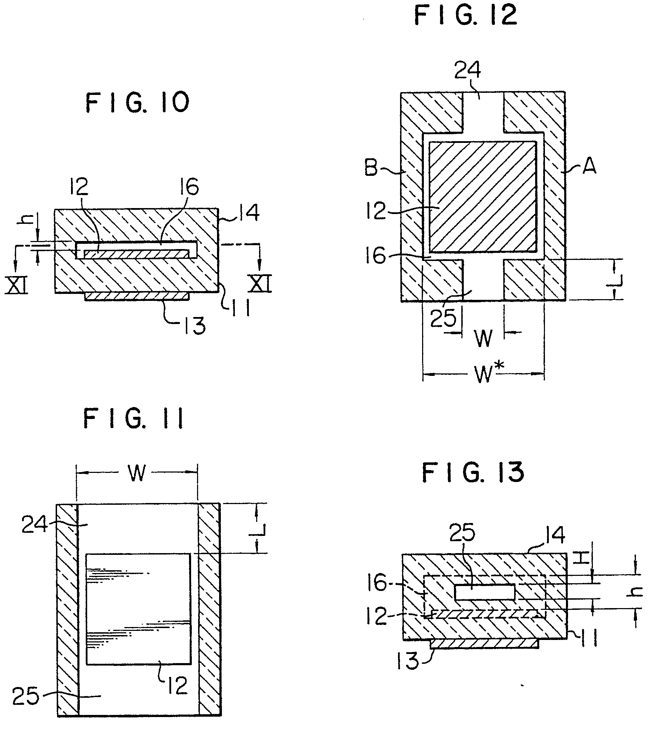

Figs. 10 and 11 illustrate the basic arrangement for application of the organic binder burning method in the production of an oxygen concentration measuring apparatus of the invention. More specifically, Fig. 10 is a front elevational view of the arrangement, while Fig. 11 is a sectional view taken along the line XI-XI of Fig. 10. This arrangement is characterized in that at least one of gas-diffusion grooves 24, 25, having a width and depth substantially equal to those of the diffusion chamber 16, is formed at both lateral sides of the electrode 12. Representing the width, length and depth of these grooves by W, L and H, respectively, the output current Ip of this sensor is about 2 times as large as that obtained by substituting L and HW for

$\ell$ and S in the formula (1) mentioned before. According to this arrangement, it is possible to obtain a small depth H of the diffusion groove 24, 25 on the order of several to several tens of micron meter ($\mu$m) by a process which will be explained later, and to increase the length of the grooves to an order of millimeters (mm). It is, therefore, possible to maintain the output critical current Ip within the oxygen pumping capacity in the first and second electrodes 12 and 13, even when the width W of the diffusion grooves is increased to an order of several millimeters (mm).

The diffusion chamber 16 and the diffusion grooves 24, 25 are formed by organic binder burning process similar to that explained before in connection with Fig. 7. Namely, after forming a layer of an organic binder by printing on the zirconia solid electrolyte 11 and the first electrode 12, a cover member 14 is placed and integrated with the zirconia electrolyte by heat- and press-bonding followed by burning. The breadth W of the gas diffusion grooves 24, 25 are substantially equal to that of the diffusion chamber 16, so that the rise of the internal gas pressure can be suppressed satisfactorily even with an appreciable rate of temperature rise during the burning.

Therefore, the undesirable effect explained before in connection with Fig. 9 such as an inflation of the diffusion chamber, pin-holes and minute cracks are eliminated regardless of any slight fluctuation in the burning conditions or factors. A sample produced by this process showed an extremely small secular change as shown by a curve c in Fig. 4.

The thickness of printed film of organic binder can be varied within the range of between several microns to about ten of micron meters ($\mu$m) in each printing step. Therefore, the depths of the gas diffusion grooves 24, 25 and the diffusion chamber 16 on the order of several tens of micron meters ($\mu$m) can be obtained by repeating the printing of the organic binder for several times. The first and the second electrodes 12 and 13 are made of platinum or its alloy and have thicknesses of about 10 micron meters ($\mu$m). From a synthetic point of view considering factors such as the overall size, yield of the product, mass-producibility and performance, the depth h of the diffusion chamber is preferably around 10 micron meters ($\mu$m), while the depth or the gas diffusion grooves 24 and 25 is about 20 micro-meters ($\mu$m) most preferably.

According to the invention, the depth of the diffusion chamber is as small as several tens of micrometers ($\mu$m) so that the internal volume Vp of the diffusion chamber can be made sufficiently small, so that a response characteristic or time constant on the order of several tens of microseconds (ms) or less, which is practically acceptable, can be obtained without substantial difficulty.

Fig. 12 shows another embodiment of the invention in which the gas diffusion grooves 24, 25 have a width W smaller than that W* of the diffusion chamber 16. This embodiment also is free from the abnormal phenomenon encountered by the conventional arrangement during the burning of the organic binder. It has been confirmed that the products are practically usable when the width ratio w/w* is not smaller than 0.1, although this condition may varies depending on the length of the gas diffusion grooves 24, 25. Although not shown, other gas-diffusion grooves orthogonal to the grooves 24 and 25 may be formed in the portions marked by A and B in Fig. 12.

Fig. 13 is a front elevational view of a still another embodiment of the oxygen concentration measuring apparatus of the invention. In this embodiment, the gas diffusion groove 25 has a depth H which is smaller than the depth h of the diffusion chamber 16. It has been confirmed that, in the case of this embodiment, practically usable products are obtained generally on condition that the depth ratio H/h is not smaller than about 0.2.

As will be understood from the foregoing description, according to the invention, the zirconia solid electrolyte and the cover member are directly bonded and integrated with each other by an effective use of the organic binder burning process, regardless of any fluctuation in the burning conditions, so that oxygen concentration measuring apparatus having superior durability are produced stably. In addition, since the internal volume of the diffusion chamber can be made sufficiently small, it is possible to obtain a high response characteristic on the order of less than several tens of microseconds (ms) in terms of time constant, even with small electric current.

## Claims

1. An oxygen concentration measuring apparatus comprising: a zirconia solid electrolyte (11), a pair of electrodes (12, 13) formed on each side of said zirconia solid electrolyte (11) and connected to a supply of electric current; a cover member (14) provided on said solid electrolyte (11) and encasing one (12) of said electrodes (12, 13); and a diffusion chamber (16) formed between said solid electrolyte (11) and said cover member (14) and provided with at least one gas diffusion groove (24, 25), **characterized** in that the solid electrolyte (11) and the cover member (14) are directly bonded and integrated with each other, and that two gas diffusion grooves (24, 25) are provided at both lateral sides of the encased electrode (12) between the solid electrolyte (11) and the cover member (14).

2. An oxygen concentration measuring apparatus according to claim 1, wherein the width (W) of said gas diffusion grooves (24, 25) is not smaller than 1/10 of the width (W*) of said gas diffusion chamber (16).

3. A method of producing an oxygen concentration measuring apparatus comprising the steps of:

forming a first electrode (12) and a second electrode (13) on each side of a plate of a solid electrolyte (11);

and attaching a cover member (14) encasing said first electrode (12) and forming a diffusion chamber (16) between said solid electrolyte (11) and said cover member, which diffusion chamber (16) is provided with a gas diffusion orifice (24, 25),

**characterized** in that

after forming said first and second electrodes (12, 13) by printing, a layer of an organic binder (19) is formed on one side of said solid electrolyte (11) so as to cover said first electrode (12);

a sheet-like cover member (14) is laminated on the same side of said solid electrolyte (11) as said first electrode (12) thus forming a laminated structure;

said laminated structure is subjected to a heat- and press-bonding at a temperature of about 200 °C;

the temperature of said laminated structure is raised at a controlled rate to gradually burn said binder (19) so as to remove said binder (19) thus forming a diffusion chamber (16) on said first electrode (12) between said cover member (14) and said solid electrolyte (11), said diffusion chamber (16) being provided with two gas diffusion grooves (24, 25) located at both lateral sides of said first electrode (12); and

said laminated structure is fired at a temperature of about 1500 °C so as to sinter said solid electrolyte (11) and said cover member (14) uniformly into one body.

4. A method according to claim 3, wherein said gas diffusion grooves (24, 25) and said gas diffusion chamber (16) are formed by said organic binder burning step.

**Revendications**

1. Dispositif de mesure de teneur en oxygène, comprenant: un électrolyte solide forme de zircone (11), un couple d'électrodes (12,13) formées sur chaque face dudit électrolyte solide formé de zircone (11) et raccordées à une alimentation en courant électrique; un élément formant capot (14) prévu sur ledit électrolyte solide (11) et enfermant l'une (12) desdites électrodes (12,13); et une chambre de diffusion (16) formée entre ledit électrolyte solide (11) et ledit élément formant capot (14) et comportant au moins une rainure (24,25) de diffusion de gaz,

caractérisé en ce que

l'électrolyte solide (11) et l'élément formant capot (4) sont réunis directement et intégrés l'un à l'autre, et que deux rainures (24,25) de diffusion de gaz sont prévues sur les deux faces latérales de l'électrode enfermée (12) entre l'électrolyte solide (11) et l'élément formant capot (14).

2. Dispositif de mesure de teneur en oxygène selon la revendication I, dans lequel la largeur (W) desdites rainures (24,25) de diffusion de gaz n'est pas inférieure à 1/10 de la largeur (W*) de ladite chambre de diffusion de gaz (16).

3. Procédé pour fabriquer un dispositif de mesure de teneur en oxygène, incluant les étapes consistant à:

former une première électrode (12) et une seconde électrode (13) sur chaque face d'une plaque d'un électrolyte solide (11); et

fixer un élément formant capot (14) enfermant ladite première électrode (12) et formant une chambre de diffusion (16) entre ledit électrolyte solide (11) et ledit élément formant capot, cette chambre de diffusion (16) comportant un orifice de diffusion de gaz (24,25),

caractérisé en ce que

après formation desdites première et seconde électrodes (12, 13) par impression, on forme une couche d'un liant organique (19) sur une face dudit électrolyte solide (11) de manière à recouvrir ladite première électrode (12);

on dispose un élément formant capot en forme de feuille (14) sur la face dudit électrolyte solide (11) où se trouve ladite première électrode (12), de manière à former ainsi une structure stratifiée;

on soumet ladite structure stratifiée à une opération de liaison par chauffage et compression à une température d'environ 200° C;

on accroît la température de ladite structure stratifiée, à une vitesse commandée, pour brûler graduellement ledit liant (19) afin d'éliminer ledit liant (19) en formant ainsi une chambre de diffusion (16) sur ladite première électrode (12) entre ledit élément formant capot (14) et ledit électrolyte solide (11), ladite chambre de diffusion (16) comportant deux rainures (24;25) de diffusion de gaz, qui sont situées sur les deux faces latérales de ladite première électrode (12); et

on fait cuire ladite structure stratifiée à une température d'environ 1500° C de manière à fritter de façon uniforme ledit électrolyte solide (11) et ledit élément formant capot (14) pour former un seul corps.

4. Procédé selon la revendication 3, selon lequel on forme lesdites rainures (24,25) de diffusion de gaz et ladite chambre (16) de diffusion de gaz au moyen de ladite étape de brûlage du liant organique.

## Ansprüche

1. Sauerstoffkonzentrations-Meßvorrichtung mit einem Zirconiumoxid-Feststoffelektrolyten (11), zwei Elektroden (12, 13), die an beiden Seiten des Zirconiumoxid-Feststoffelektrolyten (11) vorgesehen und mit einer elektrischen Stromversorgung verbunden sind, einem Gehäuseteil (14), das auf dem Feststoffelektrolyten (11) vorgesehen ist und eine der Elektroden (12) umschließt und einer Diffusionskammer (l6), die zwischen dem Feststoffelektrolyten (11) und dem Gehäuseteil (14) gebildet und mit mindestens einem Gasdiffusionskanal (24, 25) versehen ist, dadurch **gekennzeichnet**, daß der Feststoffelektrolyt (11) und das Gehäuseteil (14) direkt miteinander verbunden und miteinander integriert sind, und zwei Gasdiffusionskanäle (24, 25) an den beiden lateralen Seiten der umschlossenen Elektrode (12) zwischen dem Feststoffelektrolyten (11) und dem Gehäuseteil (14) vorgesehen sind.

2. Sauerstoffkonzentrations-Meßvorrichtung nach Anspruch 1, bei der die Breite (W) der Gasdiffusionskanäle (24, 25) nicht kleiner ist als 1/10 der Breite (W") der Gasdiffusionskammer (16).

3. Verfahren zum Herstellen einer Sauerstoffkonzentrations-Meßvorrichtung, das folgende Schritte umfaßt: Erzeugen einer ersten Elektrode (12) und einer zweiten Elektrode (13) auf den beiden Seiten einer Platte aus einem Feststoffelektrolyten (11) und

Anbringen eines Gehäuseteils (14), das die erste Elektrode (12) umschließt und eine Diffusionskammer (16) zwischen dem Feststoffelektrolyten (11) und dem Gehäuseteil (14) bildet, wobei die Diffusionskammer (16) mit einer Gasdiffusionsöffnung (24, 25) versehen ist, dadurch **gekennzeichnet**, daß

nach dem Aufbringen der ersten und zweiten Elektroden (12, 13) durch Drucken eine Schicht aus einem organischen Bindemittel (19) auf eine Seite des Feststoffelektrolyten (11) derart aufgebracht wird, daß sie die erste Elektrode (12) bedeckt,

ein flächiges Gehäuseteil (14) auf die gleiche Seite des Feststoffelektrolyten (11) wie die erste Elektrode (12) laminiert wird, wodurch eine Laminatstruktur gebildet wird,

die Laminatstruktur unter Einwirkung von Hitze und Druck bei einer Temperatur von etwa 200° C verschweißt wird,

die Temperatur der Laminatstruktur mit kontrollierter Geschwindigkeit zum allmählichen Verbrennen des Bindemittels (19) erhöht wird, so daß das Bindemittel (19) entfernt wird, wodurch eine Diffusionskammer (16) auf der ersten Elektrode (12) zwischen dem Gehäuseteil (14) und dem Feststoffelektrolyten (11) gebildet wird, wobei die Diffusionskammer (16) mit zwei Gasdiffusionskanälen (24, 25) versehen ist, die an beiden lateralen Seiten der ersten Elektrode (12) angeordnet sind, und

die Laminatstruktur auf eine Temperatur von etwa 1500° C erhitzt wird, so daß der Feststoffelektrolyt (11) und das Gehäuseteil (14) gleichmäßig zu einem Körper zusammensintern.

4. Verfahren nach Anspruch 3, wobei die Gasdiffusionskanäle (24, 25) und die Gasdiffusionskammer (16) durch den Schritt der Verbrennung des organischen Bindemittels gebildet werden.

# F I G. I

# F I G. 2

EXCESS AIR RATIO (λ)

CO, O₂ (VOLUME %)

CO

STOICH

⇨ LEAN CONTROL

O₂

RICH MIXTURE RANGE

LEAN MIXTURE RANGE

AIR/FUEL RATIO

FIG. 3

FIG. 3a

FIG. 4

# F I G. 5

# F I G. 6

## F I G. 7

## F I G. 8

## F I G. 9

# FIG. 12

# FIG. 10

# FIG. 11

# FIG. 13